# EUROPÄISCHE PATENTANMELDUNG

(11) **EP 1 320 059 A2**
(43) Veröffentlichungstag der Anmeldung: **18.06.2003**
(21) Anmeldenummer: 02026705.0
(22) Anmeldetag: 29.11.2002
(51) Int. Cl.: G06F 19/00

(54) **Rechnergestütze Verfahren zur Dokumentation eines medizinischen Befundes**

(30) Priorität: 12.12.2001 DE 10161112
(71) Anmelder: SIEMENS AKTIENGESELLSCHAFT, 80333 München (DE)
(72) Erfinder: Christ, Tilo, 91058 Erlangen (DE); Prihoda, Heinz, 90562 Heroldsberg (DE); Schmidt, Volker, Dr., 91054 Erlangen (DE); Schneider, Siegfried, Dr., 91056 Erlangen (DE); Schüll, Hans-Dieter, 91085 Weisendorf (DE); Striebel, Werner, 90592 Schwarzenbruck (DE); Zahlmann, Gudrun, Dr., 92318 Neumarkt (DE)

(57) **Zusammenfassung**

Rechnergestützte Verfahren zur Dokumentation eines medizinischen Befundes

Die Erfindung betrifft ein rechnergeschütztes Verfahren zur Dokumentation eines medizinisch unauffälligen Befundes. Beispielsweise einem Arzt (1) wird mit einer Anzeigeeinrichtung eines Rechnersystems (1a) ein für die Dokumentation geeignetes elektronisches Formular (21) angezeigt. Anschließend braucht der Arzt (1) nur ein einziges Eingabeelement (31) des Rechnersystems (1a) zu aktivieren, so dass aufgrund des aktivierten Eingabeelementes (31) das elektronische Formular (21) automatisch mit vorgegebenen, in dem Rechnersystem (1a) gespeicherten Daten, die einem medizinischen unauffälligen Befund (21a) zugeordnet sind, ausgefüllt wird. Die Erfindung betrifft ferner ein rechnergeschütztes Verfahren zur Dokumentation eines medizinischen Befundes. Für dieses Verfahren wird beispielsweise dem Arzt (1) ein für die Dokumentation geeignetes elektronisches Formulars (21) mit einer Anzeigeeinrichtung eines Rechnersystems(1a) angezeigt. Nachdem das elektronische Formular (21) mit dem medizinischen Befund zugeordneten Daten ausgefüllt ist, wird ein einziges Eingabeelemente (31) des Rechnersystems (1a) aktiviert und aufgrund des aktivierten Eingabeelementes (31) automatische wenigstens eine Handlung durch das Rechnersystem (1a) ausgeführt.

## Beschreibung

Die Erfindung betrifft Verfahren zur Dokumentation eines medizinischen Befundes mittels eines Rechnersystems.

Nach einer Untersuchung eines Patienten dokumentiert ein den Patienten untersuchender Arzt in der Regel das Ergebnis der Untersuchung beispielsweise mittels eines geeigneten Formulars. Das Ergebnis der Untersuchung wird auch als medizinischer Befund des Patienten bezeichnet. In zunehmenden Maße werden die Befunde auch elektronisch dokumentiert, indem der Arzt beispielsweise in einem Rechner gespeicherte Formulare mittels des Rechners ausfüllt. Der medizinische Befund wird auch dann dokumentiert, wenn sich aufgrund der Untersuchung herausstellt, dass der Patient gesund ist. Solch ein Befund wird auch als Normalbefund oder unauffälliger Befund bezeichnet.

Gerade bei Reihenuntersuchungen, wie Röntgenreihenuntersuchungen, Lungenreihenuntersuchungen, Mammografie-Screenings oder Augenhintergrundscreenings, Kindervorsorgeuntersuchungen oder Krebsvorsorgeuntersuchungen, ergeben sich relativ viele unauffällige Befunde, so dass der Arzt viele, im Wesentlichen inhaltsgleiche Befunde dokumentieren muss. Da die unauffälligen Befunde im Wesentlichen inhaltsgleich sind, muss der Arzt bei der Reihenuntersuchung viele Formulare im Wesentlichen gleich ausfüllen.

Nachdem der Arzt das Formular ausgefüllt hat, muss er in der Regel das den medizinischen Befund umfassende Formular ausdrucken und unterschreiben oder das im Rechner gespeicherte und ausgefüllte Formular digital signieren, das ausgefüllte Formular an weitere Personen versenden usw., was insbesondere relativ zeitaufwendig sein kann, da er mehrere Prozesse nacheinander anstoßen muss. Die eben beschriebenen Handlungen muss der Arzt im Übrigen auch bei der Dokumentation eines krankhaften Befundes, also bei einem Befund eines kranken Patienten, vornehmen.

Die Aufgabe der Erfindung ist daher, Voraussetzungen für ein erleichtertes Erstellen eines unauffälligen Befundes zu schaffen. Eine weitere Aufgabe der Erfindung ist es, Voraussetzungen für eine einfachere Verarbeitung eines unauffälligen oder krankhaften Befundes zu schaffen.

Die erste Aufgabe wird gelöst durch ein rechnergestütztes Verfahren zur Dokumentation eines unauffälligen Befundes, aufweisend folgende Verfahrensschritte:
a) Anzeigen eines für die Dokumentation geeigneten elektronischen Formulars mit einer Anzeigeeinrichtung eines Rechnersystems,
b) Aktivieren eines einzigen Eingabeelementes des Rechnersystems und
c) basierend auf dem Aktivieren des Eingabeelementes, automatisches Ausfüllen des elektronischen Formulars mit vorgegebenen, in dem Rechnersystems gespeicherten Daten, die einem unauffälligen Befund zugeordnet sind.

Wie obenstehend bereits beschrieben, füllt der Arzt bei einer Dokumentation eines medizinischen Befundes in der Regel ein vorgefertigtes, der Art der Untersuchung zugeordnetes Formular aus, welches z.B. die Form einer Checkliste hat, so dass der Arzt alle für den medizinischen Befund notwendigen Fragen beantwortet. Bei unauffälligen Befunden verschiedener Patienten, bei denen zumindest ähnliche Untersuchungen durchgeführt wurden, sind diese Formulare und die Beantwortung der Fragen der Formulare im Wesentlichen gleich, so dass der Arzt viele Formulare im Wesentlichen gleich ausfüllen muss. Will nun der Arzt ein solches Formular ausfüllen, wobei das Formular einem Patienten mit einem unauffälligen Befund zugeordnet ist, braucht er erfindungsgemäß nur das Eingabeelement zu aktivieren und die Fragen des Formulars werden mit in dem Rechnersystem gespeicherten Daten beantwortet. Folglich braucht der Arzt beim Erstellen des unauffälligen Befundes nur ein Eingabeelement zu aktivieren, um das ausgefüllte Formular zu erhalten, das dem unauffälligen Befund entspricht. Ein zeitaufwändiges im Wesentlichen identisches Ausfüllen mehrerer Formulare entfällt somit.

Gemäß einer Ausführungsform der Erfindung ist vorgesehen, dass in das Formular ein Fenster und/oder ein Feld eingeblendet ist, aufgrund dessen das Aktivieren des Eingabeelementes aufgefordert wird. Wenn der Arzt mit der Anzeigeeinrichtung des Rechnersystems das Formular betrachtet, kann das Fensters oder das Feld in das dargestellte Formular oder außerhalb des dargestellten Formulars eingeblendet werden.

Nach einer weiteren Variante der Erfindung ist das Eingabeelement eine Rechnermaus oder eine Tastatur des Rechnersystems und das Aktivieren des Eingabeelementes ein Drücken einer Taste der Rechnermaus oder der Tastatur. Daher kann gemäß einer besonders bevorzugten Variante der Erfindung das Feld mit der Rechnermaus des Rechnersystems angeklickt und dadurch das Formular automatisch mit den vorgegebenen und in dem Rechnersystem gespeicherten Daten ausgefüllt werden. Das Fenster zur Aufforderung zum Aktivieren des Eingabeelements kann auch ein Feld umfassen, das insbesondere durch Anklicken das automatische Ausfüllen des Formulars initiiert.

Aufgrund des Aktivierens des Eingabeelementes wird gemäß einer weiteren Ausführungsform der Erfindung wenigstens eine weitere vorgegebene Handlung von dem Rechnersystem automatisch ausgeführt. Die weitere Handlung ist gemäß einer besonders bevorzugten Variante der Erfindung ein digitales Signieren des ausgefüllten elektronischen Formulars, eine Verschlüsselung des ausgefüllten elektronischen Formulars, ein Übermitteln des ausgefüllten elektronischen Formulars an wenigstens einen Empfänger, ein Archivieren des ausgefüllten elektronischen Formulars, eine Rechnungserstellung oder eine Abbuchung von einem Konto. Die eben genannten Handlungen werden oft nach dem Erstellen eines medizinischen Befundes, also auch nach dem Erstellen eines unauffälligen Befundes durchgeführt. Aufgrund des erfindungsgemäßen Verfahrens brauchen diese Handlungen aber nicht einzeln initiiert werden, sondern werden automatisch aufgrund des Aktivieren des Eingabeelementes ausgeführt.

Die zweite Aufgabe der Erfindung wird gelöst durch ein rechnergestütztes Verfahren zur Dokumentation eines medizinischen Befundes, aufweisend folgende Verfahrensschritte:
a) Anzeigen eines für die Dokumentation geeigneten elektronischen Formulars mit einer Anzeigeeinrichtung eines Rechnersystems,
b) Ausfüllen des elektronischen Formulars mit dem medizinischen Befund zugeordneten Daten,
c) Aktivieren eines einzigen Eingabeelementes des Rechnersystems und
d) aufgrund des Aktivierens des Eingabeelementes, automatisches Ausführen wenigstens einer Handlung durch das Rechnersystem.

Wie obenstehend bereits beschrieben, muss ein Arzt nach dem Erstellen eines medizinischen Befundes oft mehrere weitere Handlungen, wie z.B. ein digitales Signieren des ausgefüllten elektronischen Formulars, eine Verschlüsselung des ausgefüllten elektronischen Formulars, ein Übermitteln des ausgefüllten elektronischen Formulars an wenigstens einen Empfänger, ein Archivieren des ausgefüllten elektronischen Formulars, eine Rechnungserstellung oder eine Abbuchung von einem Konto, durchführen. Durch das Aktivieren eines einzigen Eingabeelementes werden diese Handlungen erfindungsgemäß automatisch ausgeführt, wodurch dem Arzt oder der Person, die das dem medizinischen Befund zugeordnete elektronische Formular ausfüllt, Zeit gespart wird.

Je nach dem, ob der Befund ein unauffälliger oder ein krankhafter Befund ist, kann es sein, dass verschiedene Handlungen ausgeführt werden müssen. So kann es z.B. sein, dass nur bei einem krankhaften Befund ein Gesundheitsamt oder Spezialisten zur Weiterbehandlung des dem krankhaften Befund zugeordneten Patienten verständigt werden müssen. Dies kann z.B. bei einer Reihenlungenuntersuchung der Fall sein. Daher ist gemäß einer bevorzugten Ausführungsform der Erfindung vorgesehen, dass überprüft wird, ob die dem Befund zugeordneten Daten einem unauffälligen oder einem krankhaften Befund zugeordnet sind und entsprechend der Überprüfung wenigstens eine unterschiedliche Handlung automatisch ausgeführt wird.

Nach einer Ausführungsform der Erfindung ist das Eingabeelement eine Rechnermaus oder eine Tastatur des Rechnersystems und das Aktivieren des Eingabeelementes ein Drücken einer Taste der Rechnermaus oder der Tastatur. Ferner ist gemäß einer weiteren Variante der Erfindung vorgesehen, dass in das elektronische Formular ein Fenster und/oder ein Feld eingeblendet wird, aufgrund dessen das Aktivieren des Eingabeelementes aufgefordert wird. So kann gemäß einer besonders bevorzugten Ausführungsform der Erfindung das Feld mit der Rechnermaus angeklickt und dadurch die Handlung ausgeführt werden. Die Handlung kann also durch einen einzigen Rechnermausklick initiiert werden.

Ein Vorteil der erfindungsgemäßen Verfahren ist, dass komplexe Vorgänge insbesondere mit einem Mausklick oder einem Drücken einer einzigen Taste erledigt werden können. Ferner kann es vorteilhaft sein, wenn Informationen in mehreren Fenstern gleichzeitig dargestellt werden, so dass ein Anwender, also z.B. der den medizinischen Befund erstellende Arzt, für den eigentlichen Bestätigungsvorgang alle erforderlichen Daten sieht. Das ist beispielsweise für das digitale Signieren nötig, da das zu Signierende als Ganzes angezeigt werden muss.

Die erfindungsgemäßen Verfahren können insbesondere bei jeder Form der elektronischen Befundung, bei telemedizinischen Dienstleistungen, für eine sogenannte Second Opinion, bei einem Screening oder bei institutsinternen Informationssystemen verwendet werden.

Aufgrund der erfindungsgemäßen Verfahren ergibt sich eine deutliche Beschleunigung des Dokumentationsvorgangs.

Ausführungsbeispiele sind exemplarisch in den beigelegten schematischen Zeichnungen dargestellt. Es zeigen:
- Fig. 1: ein die erfindungsgemäßen Verfahren veranschaulichendes Szenario,
- Fig. 2: ein elektronisches Formular,
- Fig. 3: Eine Rechnertastatur,
- Fig. 4: ein ausgefülltes elektronisches Formular, das einen unauffälligen Befund dokumentiert, und
- Fig. 5: ein ausgefülltes elektronisches Formular, das einen krankhaften Befund dokumentiert.

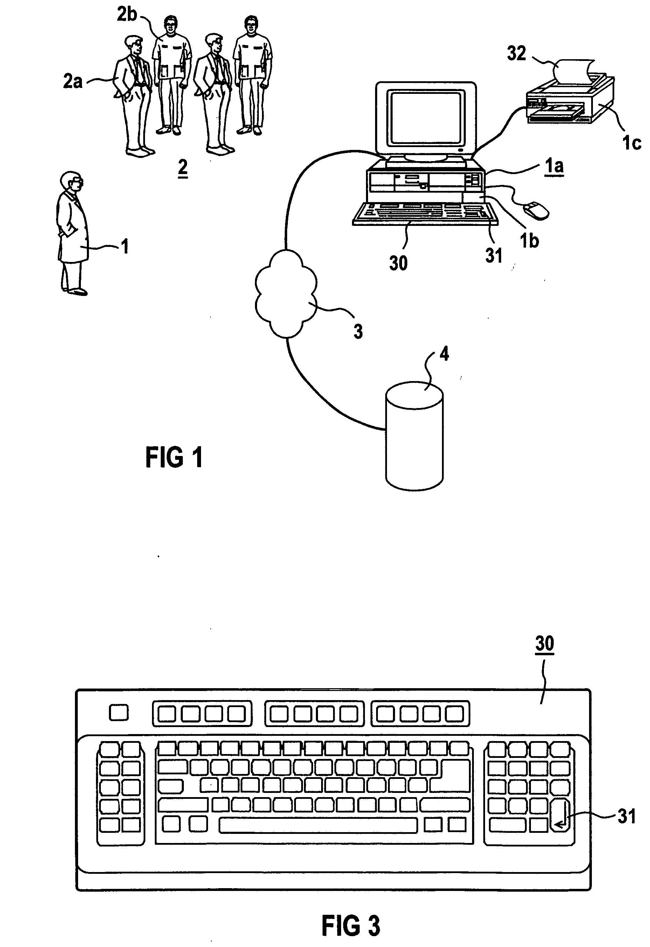

Die Figur 1 zeigt einen Arzt 1, der eine Lungenreihenuntersuchung mehrerer Patienten 2 durchführt. Für die Lungenreihenuntersuchung erstellt der Arzt 1 von jedem der Patienten 2 jeweils ein in der Figur 1 nicht dargestelltes Röntgenbild mit einem ebenfalls in der Figur 1 nicht dargestellten Röntgengerät. Außerdem führt der Arzt 1 von jedem der Patienten 2 eine Blutuntersuchung durch.

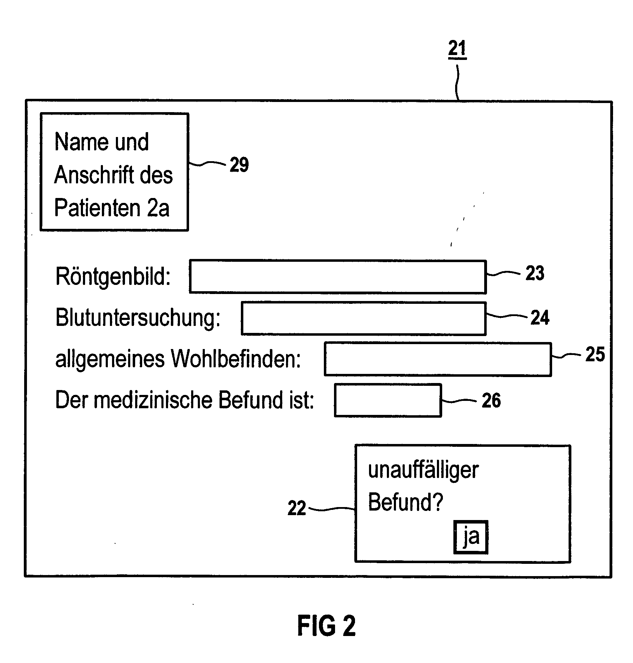

Um das Untersuchungsergebnis eines jeden der Patienten 2 zu dokumentieren, erstellt der Arzt 1 für jeden der Patienten 2 einen medizinischen Befund. Die medizinischen Befunde fertigt der Arzt 1 im Falle des vorliegenden Ausführungsbeispiels mit einem Rechner 1a an. In dem Rechner 1a ist dazu ein geeignetes Rechnerprogramm gespeichert, welches derart ausgeführt ist, dass dem Arzt 1 ein elektronisches Formular 21, welches in der Figur 2 dargestellt ist, bei Aufruf des Rechnerprogrammes auf dem Bildschirm des Rechners 1a angezeigt wird. Das elektronische Formular 21 umfasst ein Feld 29, in das der Arzt 1 den Namen und die Anschrift desjenigen Patienten der Patienten 2 einträgt, für den er gerade einen medizinischen Befund erstellen möchte. Das elektronische Formular 21 umfasst ferner Felder 23, 24 und 25, in die der Arzt 1 eine eventuelle Auffälligkeit des Röntgenbildes, der Blutuntersuchung und des allgemeinen Wohlbefindens des Patienten, für den der medizinische Befund erstellt wird, einträgt. Aufgrund dieser Eintragungen kann der Arzt 1 entscheiden, ob der medizinische Befund unauffällig oder krankhaft ist, was er in das Feld 26 einträgt.

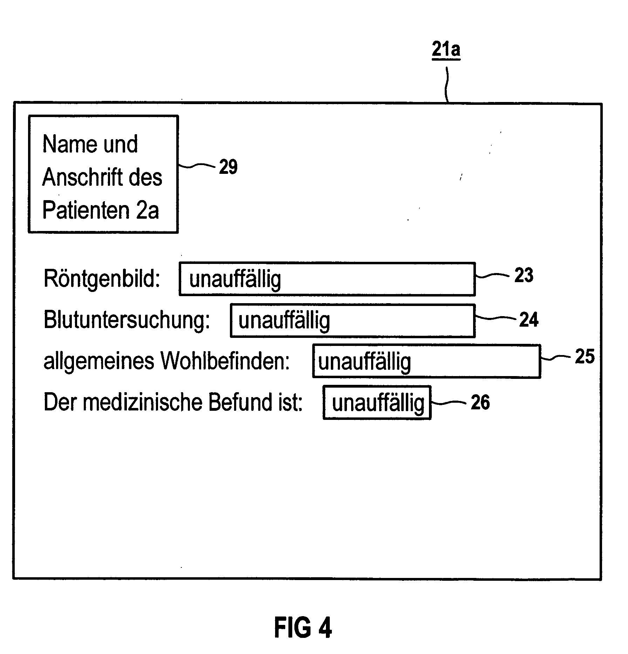

Im Falle des vorliegenden Ausführungsbeispiels erstellt der Arzt 1 einen medizinischen Befund für einen Patienten 2a der Patienten 2. Nachdem der Arzt 1 den Namen und die Anschrift des Patienten 2a in das elektronische Formular 21 eingetragen hat, veranlasst das Rechnerprogramm, dass in das Formular 21 automatisch ein Feld 22 eingeblendet wird, mittels dessen der Arzt 1 gefragt wird, ob es sich bei dem vorliegenden medizinischen Befund um einen unauffälligen Befund handelt. Im Falle des vorliegenden Ausführungsbeispiels kommt der Arzt 1 aufgrund des von dem Patienten 2a angefertigten Röntgenbildes und der von dem Patienten 2a durchgeführten Blutuntersuchung zu dem Schluss, dass der Patient 2a gesund ist, ihm also ein unauffälliger Befund zuzuordnen ist. Daher drückt der Arzt 1 die "Eingabetaste" Taste 31 der Tastatur 30 des Rechners 1a. Die Tastatur 30 ist in der Figur 3 näher dargestellt.

Nach Drücken der "Eingabe" Taste 31 veranlasst das Rechnerprogramm, dass das elektronische Formular 21 automatisch derart ausgefüllt wird, dass ein unauffälliger Befund des Patienten 2a dokumentiert wird. Das Rechnerprogramm füllt daher automatisch die Felder 23, 24, 25 und 26 mit dem Wort "unauffällig" aus, wie dies in der Figur 4 gezeigt ist. Das ausgefüllte elektronische Formular ist in der Figur 4 mit dem Bezugszeichen 21a versehen.

Mit Drücken der "Eingabe"-Taste 31 der Tastatur 30 des Rechners 1a füllt das Rechnerprogramm nicht nur das elektronische Formular 21 automatisch aus; das Rechnerprogramm versieht auch noch das ausgefüllte elektronische Formular 21a automatisch mit einer elektronischen Signatur des Arztes 1, verschlüsselt das ausgefüllte elektronische Formular 21a mit im Allgemeinen bekannten Verfahren, speichert das verschlüsselte ausgefüllte elektronische Formular 21a auf einem Speicher 1b des Rechners 1a und übermittelt es automatisch an eine zentrale Datenbank 4, die im Falle des vorliegenden Ausführungsbeispieles einem Gesundheitsamt zugeordnet ist. Damit das ausgefüllte elektronische Formular 21a an die Datenbank 4 übermittelt werden kann, ist im Falle des vorliegenden Ausführungsbeispieles der Rechner 1a und die Datenbank 4 an ein Informationsfernübertragungsnetz 3 angeschlossen. Außerdem veranlasst das Rechnerprogramm automatisch, dass ein an den Rechner 1a angeschlossener Drucker 1c eine Rechnung 32 für die Erstellung des unauffälligen Befundes des Patienten 2a druckt. Die Rechnung 32 ist für die Krankenkasse des Patienten 2a bestimmt.

Danach möchte der Arzt 1 einen medizinischen Befund für einen Patienten 2b der untersuchten Patienten 2 erstellen. Daher ruft er ein neues unausgefülltes elektronisches Formular 21 auf und trägt in das Feld 29 den Namen und die Anschrift des Patienten 2b ein. Danach blendet das Rechnerprogramm wieder automatisch das Feld 22 in das elektronische Formular 21 ein.

Im Falle des vorliegenden Ausführungsbeispiels weist das Röntgenbild des Patienten 2b einen Schatten auf. Außerdem klagt der Patient 2b über Brustschmerzen. Der Arzt will daher einen krankhaften Befund ausstellen.

Daher drückt der Arzt 1 nicht die "Eingabe"-Taste 31 der Tastatur 30 des Rechners 1a, sondern füllt das Feld 23 mit dem Kommentar "Schatten am linken Lungenflügel" aus. Daraufhin blendet das Rechnerprogramm automatisch das Feld 22 aus. Da im Falle des vorliegenden Ausführungsbeispiels die Blutuntersuchung des Patienten 2b unauffällig war, füllt der Arzt 1 das Feld 24 mit dem Wort "unauffällig" aus. Um die Brustschmerzen des Patienten 2b zu dokumentieren, füllt der Arzt 1 das Feld 25 mit dem Kommentar "Schmerzen in der Brust" aus. Ferner füllt der Arzt 1 das Feld 26 mit dem Wort "krankhaft" aus, um somit den krankhaften Befund des Patienten 2b zu dokumentieren.

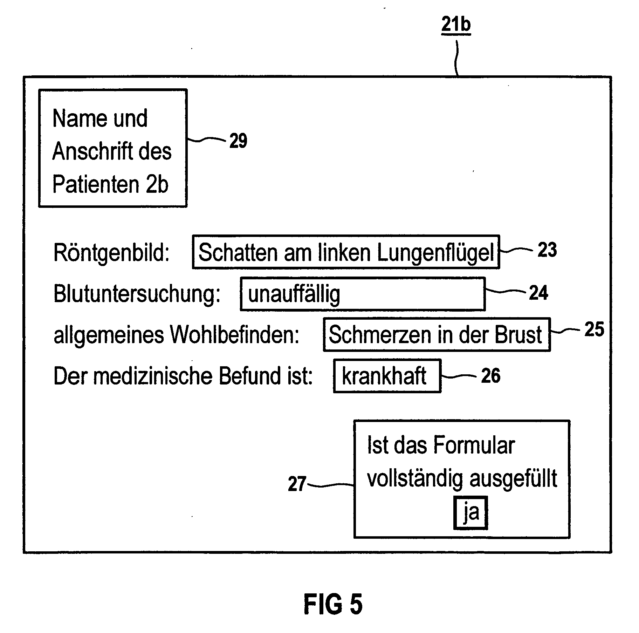

Nachdem der Arzt 1 die Felder 23, 24, 25 und 26 ausgefüllt hat, blendet das Rechnerprogramm automatisch ein Feld 27 in das ausgefüllte elektronische Formular ein. Das ausgefüllte elektronische Formular ist in der Figur 5 dargestellt und mit dem Bezugszeichen 21b versehen. Das Feld 27 fragt den Arzt 1, ob er das elektronische Formular 21 vollständig ausgefüllt hat. Da der Arzt 1 keine Korrekturen an dem ausgefüllten elektronischen Formular 21b vornehmen möchte, drückt er die "Eingabe"-Taste 31 der Tastatur 30 des Rechners 1a.

Daraufhin versieht das Rechnerprogramm das ausgefüllte elektronische Formular 21b automatisch mit einer elektronischen Signatur des Arztes 1, verschlüsselt das ausgefüllte elektronische Formular 21b, speichert das verschlüsselte ausgefüllte elektronische Formular 21b auf dem Speicher 1b des Rechners 1a und übermittelt es automatisch an die zentrale Datenbank 4 des Gesundheitsamts. Außerdem veranlasst das Rechnerprogramm automatisch, dass der Drucker 1c eine Rechnung für die Erstellung des krankhaften Befundes des Patienten 2b druckt.

Die Rechnung ist für die Krankenkasse des Patienten 2b bestimmt.

Die beschriebenen Ausführungsbeispiele sind im Übrigen nur exemplarisch zu verstehen.

## Patentansprüche

1. Rechnergeschütztes Verfahren zur Dokumentation eines unauffälligen Befundes, aufweisend folgende Verfahrensschritte:
a) Anzeigen eines für die Dokumentation geeigneten elektronischen Formulars (21) mit einer Anzeigeeinrichtung eines Rechnersystems (1a),
b) Aktivieren eines einzigen Eingabeelementes (31) des Rechnersystems (1a) und
c) aufgrund des Aktivierens des Eingabeelementes (31), automatisches Ausfüllen des elektronischen Formulars (21) mit vorgegebenen, in dem Rechnersystem (1a) gespeicherten Daten, die einem unauffälligen Befund (21a) zugeordnet sind.

2. Verfahren nach Anspruch 1, bei dem in das elektronische Formular (21) ein Feld und/oder ein Fenster (22) eingeblendet ist, aufgrund dessen das Aktivieren des Eingabeelementes (31) aufgefordert wird.

3. Verfahren nach Anspruch 1 oder 2, bei dem das Eingabeelement (31) eine Rechnermaus oder eine Tastatur (30) des Rechnersystems (1a) und das Aktivieren des Eingabeelementes (31) ein Drücken einer Taste (31) der Rechnermaus oder der Tastatur (30) ist.

4. Verfahren nach Anspruch 3, bei dem das Feld mit der Rechnermaus des Rechnersystems angeklickt wird und dadurch das elektronische Formular (21) automatisch mit den vorgegebenen und in dem Rechnersystem (1a) gespeicherten Daten ausgefüllt wird.

5. Verfahren nach einem der Ansprüche 1 bis 4, bei dem aufgrund des Aktivierens des Eingabeelementes (31) wenigstens eine weitere vorgegebene Handlung von dem Rechnersystem (1a) automatisch ausgeführt wird.

6. Verfahren nach Anspruch 5, bei dem die weitere Handlung ein digitales Signieren des ausgefüllten elektronischen Formulars (21a), eine Verschlüsselung des ausgefüllten elektronischen Formulars (21a), ein Übermitteln des ausgefüllten elektronischen Formulars (21b) an wenigstens einen Empfänger (4), ein Archivieren des ausgefüllten elektronischen Formulars (21a), eine Rechnungserstellung (32) oder eine Abbuchung von einem Konto ist.

7. Rechnergeschütztes Verfahren zur Dokumentation eines medizinischen Befundes, aufweisend folgende Verfahrensschritte:
a) Anzeigen eines für die Dokumentation geeigneten elektronischen Formulars (21) mit einer Anzeigeeinrichtung eines Rechnersystems(1a),
b) Ausfüllen des elektronischen Formulars (21) mit dem medizinischen Befund zugeordneten Daten,
c) Aktivieren eines einzigen Eingabeelementes (31) des Rechnersystems (1a) und
d) aufgrund des Aktivierens des Eingabeelementes (31), automatisches Ausführen wenigstens einer Handlung durch das Rechnersystem (1a).

8. Verfahren nach Anspruch 7, bei dem die Handlung ein digitales Signieren des ausgefüllten elektronischen Formulars (21b), eine Verschlüsselung des ausgefüllten elektronischen Formulars (21b), ein Übermitteln des ausgefüllten elektronischen Formulars (21b) an wenigstens einen Empfänger (4), ein Archivieren des ausgefüllten elektronischen Formulars (21b), eine Rechnungserstellung oder eine Abbuchung von einem Konto ist.

9. Verfahren nach Anspruch 7 oder 8, bei dem überprüft wird, ob die dem Befund zugeordneten Daten einem unauffälligen oder einem krankhaften Befund zugeordnet sind und entsprechend der Überprüfung wenigstens eine unterschiedliche Handlung automatisch ausgeführt wird.

10. Verfahren nach einem der Ansprüche 7 bis 9, bei dem das Eingabeelement (31) eine Rechnermaus oder eine Tastatur (30) des Rechnersystems (1a) und das Aktivieren des Eingabeelementes (31) ein Drücken einer Taste (31) der Rechnermaus oder der Tastatur (30) ist.

11. Verfahren nach einem der Ansprüche 7 bis 10, bei dem in das elektronische Formular (21) ein Feld und/oder ein Fenster (27) eingeblendet wird, aufgrund dessen das Aktivieren des Eingabeelementes (31) aufgefordert wird.

12. Verfahren nach Anspruch 11, bei dem das Feld mit der Rechnermaus angeklickt wird und dadurch die Handlung ausgeführt wird.
